# EUROPEAN PATENT APPLICATION

(11) **EP 1 382 292 A1**
(43) Date of publication of application: **21.01.2004**
(21) Application number: 03007272.2
(22) Date of filing: 31.03.2003
(51) Int. Cl.: A61B 5/00

(54) **Medical-information providing apparatus and portable telephone**

(30) Priority: 17.07.2002 JP 2002208008
(71) Applicant: Colin Corporation, Komaki-shi, Aichi-ken (JP)
(72) Inventor: Shinoda, Masayuki, Komaki-shi, Aichi-ken (JP)
(74) Representative: Winter, Brandl, Fürniss, Hübner, Röss, Kaiser, Polte Partnerschaft

(57) **Abstract**

A medical-information providing apparatus comprising a physical-information sensor (40) which is directly or indirectly connectable to a portable telephone (10) operable by a registered member, and obtains physical information from the registered member; a memory device (28, 64) to which the portable telephone is connectable via a communication line (16) and which stores, for the registered member, the physical information obtained by the physical-information sensor; and a medical-information providing device (68) which provides, to the portable telephone operated by the registered member, medical information corresponding to the physical information stored for the registered member by the memory device.

## Description

### BACKGROUND OF THE INVENTION

### Field of the Invention

The present invention relates to a medical-information providing apparatus which can perform medical consultation or physical checkup based on physical information obtained from a member and provide, to the member, reliable medical information provided by a medical worker (e.g., a doctor), as needed.

### Related Art Statement

When a living person wants to undergo a periodic, physical checkup for preventing diseases, cares about health, or is in bad shape, the person needs to visit a medical treatment facility, such as a hospital or a doctor's office, and request a specialist or a practitioner to check up his or her body and make a diagnosis. For example, when a family wants to keep their health and thereby keep their happiness, preventive medicine and early treatment are important. Therefore, it is desirable to provide an apparatus or a system which can quickly and easily provide medical information to a person when the person needs it.

However, in many cases, people must wait for two or three hours at medical treatment facilities, such as hospitals or doctor's offices, and accordingly must absent themselves from their business. In addition, people must see actual medical workers, such as specialists or practitioners, for requesting them to check up their bodies. Thus, seeing a medical worker is time-consuming and costs high.

### SUMMARY OF THE INVENTION

It is therefore an object of the present invention to provide a medical-information providing apparatus which can quickly or economically perform medical consultation via a communication line.

The above object has been achieved by the present invention. According to a first aspect of the present invention, there is provided a medical-information providing apparatus comprising a physical-information sensor which is directly or indirectly connected to a portable telephone that is operated by a registered member, and which obtains physical information from the registered member; a memory device to which the portable telephone is connected via a communication line and which stores, for the registered member, the physical information obtained by the physical-information sensor; and a medical-information providing device which provides, to the registered member, medical information corresponding to the physical information stored for the registered member by the memory device.

According to this aspect, when the physical information obtained by the physical-information sensor directly or indirectly connected to the portable telephone of a living person registered as a member is sent via the telephone, the memory device stores the physical information for the member, and sends, to the member, medical information corresponding to the physical information stored for the member. Thus, even if the member may reside at a remote place, the member can automatically obtain an evaluation or a diagnosis made on the physical information. That is, the member can quickly or economically undergo a medical consultation via the communication line. In addition, since the medical information can indicate a sign of a serious disease, preventive medicine can be promoted. Since the member can quickly and easily obtain medical information, as needed, preventive medicine or early treatment can be improved, and a healthy or happy family can be easily kept.

According to a preferred feature of the first aspect of the present invention, the physical-information sensor comprises a pulse-wave sensor which is adapted to be pressed against a skin of the registered member so as to detect a pulse wave produced by an artery present under the skin, and the apparatus further comprises a pulse-wave displaying means for operating a display device of the portable telephone to display the pulse wave detected by the pulse-wave sensor. According to this feature, since the pulse wave detected by the pulse-wave sensor connected directly or indirectly to the portable telephone is continuously displayed on the display device of the telephone, the member can easily adjust, while viewing the pulse wave displayed on the display device, the position at which the sensor detects the pulse wave, or the pressure with which the sensor is pressed.

According to another feature of the first aspect of the present invention, the medical-information providing apparatus further comprises a medical-information data base which stores a plurality of sets of medical information corresponding to a plurality of sets of physical information obtained from a plurality of registered members, respectively; and a medical-information retrieving means for retrieving, from the sets of medical information stored by the medical-information data base, a set of medical information to be provided to the registered member, based on a set of physical information obtained from the registered member by the physical-information sensor, and the medical-information providing device provides, to the portable telephone operated by the registered member, the set of medical information retrieved by the medical-information retrieving means. According to this feature, the medical information needed by the member can be automatically and economically provided to the member.

According to another feature of the first aspect of the present invention, the medical-information providing apparatus further comprises a physical-information-abnormality judging means for judging whether the physical information sent from the portable telephone operated by the registered member falls outside a predetermined reference range, and thereby judging whether the physical information is abnormal; a medical-worker selecting means for selecting, when the physical-information-abnormality judging means judges that the physical information is abnormal, one of a plurality of terminal devices, and thereby selecting one of a plurality of registered medical workers who operate the plurality of terminal devices, respectively; and a sending and receiving means for sending the physical information judged as being abnormal by the physical-information-abnormality judging means, to said one terminal device operated by said one medical worker selected by the medical-worker selecting means, requesting said one medical worker to make a diagnosis on the physical information judged as being abnormal, and receiving the diagnosis made by said one medical worker and sent from said one terminal device, and the medical-information providing device provides, to the portable telephone operated by the registered member, the set of medical information comprising the diagnosis sent from said one terminal device operated by said one medical worker and received by the sending and receiving means. According to this feature, when the physical-information-abnormality judging device judges that the physical information is abnormal, the medical-worker selecting device selects one of the respective terminal devices of the registered medical workers, and the sending and receiving device sends the physical information judged as being abnormal, to the terminal device of the medical worker selected by the medical-worker selecting device, requests the selected medical worker to make a diagnosis on the physical information, and receives the diagnosis made by the medical worker. The medical-information providing device sends the diagnosis to the portable telephone of the member. Thus, when the physical information is abnormal, the member can quickly obtain reliable medical information provided by a medical worker such as a doctor.

According to another feature of the first aspect of the present invention, the medical-worker selecting means selects said one medical worker based on a sort of the physical information sent from the registered member and judged as being abnormal by the physical-information-abnormality judging means. According to this feature, the medical-worker selecting means selects a medical worker appropriate for the physical information judged as being abnormal, e.g., a specialist with respect to the field related to the abnormal physical information. Thus, when the physical information is abnormal, the member can obtain highly reliable medical information.

According to another feature of the first aspect of the present invention, the medical-worker selecting means selects said one medical worker based on an address of the registered member who has sent the physical information to be judged as being abnormal by the physical-information-abnormality judging means. According to this feature, the medical-worker selecting means selects a medical worker appropriate for the address of the member, for example, a doctor who belongs to a medical treatment facility located in an area where the member can go from his or her address. Therefore, when the physical information is abnormal, the member can go to see the doctor who has made the diagnosis on the abnormal physical information.

According to another feature of the first aspect of the present invention, the medical-information providing apparatus further comprises a charging means for charging the registered member operating the portable telephone, for the diagnosis sent by said one terminal device operated by said one medical worker and provided by the medical-information providing device to the portable telephone operated by the registered member. According to this feature, only the member who receives the diagnosis made on the abnormal physical information by the medical worker, i.e., only the beneficiary pays the cost needed for obtaining the additional diagnosis, and accordingly a subscription fee that each member must pay for routinely obtaining evaluations or diagnoses automatically made on respective pieces of physical information by the medical-information data base, i.e., a so-called virtual health secretary or virtual hospital, that is, for usually using an e-health-care-network, can be reduced. Thus, usually, each member can economically obtain a diagnosis made on physical information.

According to a second aspect of the present invention, there is provided a portable telephone for being operated by a member registered by a medical-information providing system, the telephone comprising a physical-information sensor which obtains physical information from the registered member; a diagnosing device which automatically makes a diagnosis on the registered member based on the physical information obtained by the physical-information sensor; and a sending device which sends the physical information obtained by the physical-information sensor and the diagnosis made by the diagnosing device, to the medical-information providing system, so that the medical-information providing system provides medical information to the portable telephone.

According to this aspect, the diagnosing device makes a diagnosis on the member based on the physical information obtained by the physical-information sensor of the portable telephone, and the sending device sends the physical information and the diagnosis made by the diagnosing device, to the medical-information providing system, so that the medical-information providing system provides medical information to the portable telephone. Thus, the medical-information providing system provides the medical information corresponding to the physical information and the diagnosis made by the diagnosing device. Therefore, even if the member may reside at a remote place, the member can automatically obtain an evaluation or a diagnosis made on the physical information. That is, the member can quickly or economically undergo a medical consultation via the communication line. In addition, since the medical information can indicate a sign of a serious disease, preventive medicine can be promoted.

According to a preferred feature of the second aspect of the present invention, the physical-information sensor is adapted to be directly or indirectly connected to a connection potion of the portable telephone and comprises at least one of a pressure-pulse-wave sensor which is adapted to be pressed against the registered member so as to detect a pressure pulse wave produced by an artery of the member; a photoelectric-pulse-wave sensor; and a blood-oxygen-saturation sensor.

According to another feature of the second aspect of the present invention, the diagnosing means diagnoses, based on the physical information obtained by the physical-information sensor, the registered member with at least one of arrhythmia, lowering of peripheral blood circulation, and decreasing of blood oxygen saturation.

Thus, the medical information may comprise a pressure pulse wave, a photoelectric pulse wave, or a blood oxygen saturation, and a diagnosis such as arrhythmia, condition of peripheral blood circulation, or decreasing of blood oxygen saturation.

According to another feature of the second aspect of the present invention, the physical-information sensor is adapted to be fixed on a skin of the registered member so as to obtain the physical information from a portion below the skin, and sends a detection signal representing the obtained physical information, to the portable telephone, and the diagnosing means makes the diagnosis on the registered member based on the physical information sent by the physical-information sensor. According to this feature, the physical-information sensor can be separated from the portable telephone, and be fixed on the skin of the member so as to obtain the physical information from the portion below the skin, and sends the detection signal representing the obtained physical information, to the portable telephone. Thus, the degree of freedom with respect to the position where the physical-information sensor obtains the physical information can be improved.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a medical-information providing system as a medical-information providing apparatus to which the present invention is applied, the medical-information providing system utilizing a communication line;
Fig. 2 is a side elevation view of a sensor that is connected to a portable telephone shown in Fig. 1;
Fig. 3 is a side elevation view of a press surface of the sensor directly connected to the portable telephone shown in Fig. 1;
Fig. 4 is a view showing a state in which the sensor directly connected to the portable telephone shown in Fig. 1 is pressed against a radial artery;
Fig. 5 is a view showing a state in which the sensor directly connected to the portable telephone shown in Fig. 1 is pressed against a carotid artery;
Fig. 6 is a view showing a state in which the sensor connected via an electric line to the portable telephone shown in Fig. 1 is pressed against a carotid artery;
Fig. 7 is a block diagram for explaining essential control functions of a server shown in Fig. 1;
Fig. 8 is a view showing an example of a set of medical information that is provided by the server of Fig. 1 to a member; and
Fig. 9 is a flow chart representing the essential control functions of the server of Fig. 1, i.e., a virtual-hospital controlling routine.

### DETAILED DESCRIPTION OF PREFERRED EMBODIMENTS

Hereinafter, there will be described a preferred embodiment of the present invention in detail by reference to the drawings.

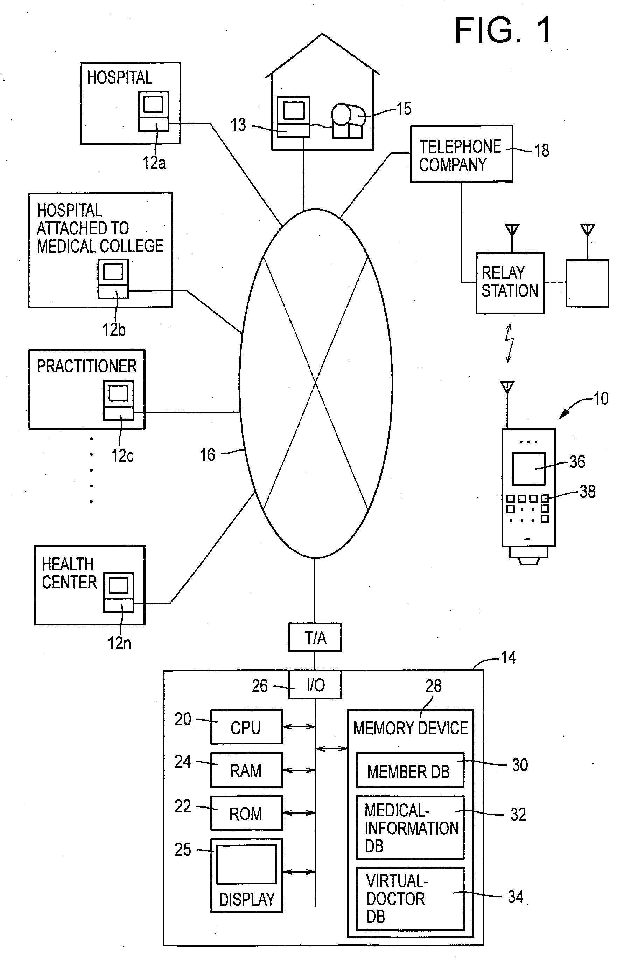

Fig. 1 is a view for explaining the construction of a medical-information providing system as a medical-information providing apparatus to which the present invention is applied. As shown in Fig. 1, the present medical-information providing system includes a plurality of medical worker's terminal devices 12a, 12b, 12c, ..., 12n that are provided in respective medical treatment facilities such as a hospital, a hospital attached to a medical college, a practitioner, or a health center, and are operable by respective medical workers such as a doctor; and a server 14 as a virtual hospital that is provided in an information providing company. The present system further includes a communication line 16, such as a wire or wireless internet (i.e., a communication network) or a wire or wireless telephone line, that connects the medical worker's terminal devices 12n and the server 14 with each other, so that highly secret codes, such as SSL (secure sockets layer), can be communicated between those elements 12n, 14. Moreover, the present system includes a plurality of portable telephones 10 as member's terminal devices that have the function of making communications via an internet and are operable by individual members. Each of the portable telephones 10 can communicate, via a telephone company 18 and the communication line 16, with each of the medical worker's terminal devices 12a, 12b, 12c, ..., 12n, and with the server 14 of the information providing company that functions as the virtual hospital. Furthermore, the present system includes a terminal device 13 that is provided in a member's house, and a physical-information obtaining device 15 that is connected to the terminal device 13 and obtains physical information such as blood pressure, from a member who owns the house. The terminal device 13 sends the physical information to the server 14. The medical worker's terminal devices 12a, 12b, 12c, ..., 12n are provided by, e.g., large-size computers, and the server 14 is provided by, e.g., a high-speed and high-capacity electronic computer.

The server 14 is essentially provided by a so-called microcomputer including a CPU (central processing unit) 20, a ROM (read only memory) 22 that stores control programs, a RAM (random access memory) 24 that functions as a temporary storage device, a display device 25, and an input-and-output interface 26 that is connected via a terminal adaptor TA to the communication line 16. The server 14 includes a memory device 28 that stores various sorts of data bases (DB) corresponding to various sorts of information. More specifically described, the memory device 28 includes a member DB (data base) 30 that stores respective names of a plurality of living persons who have contracted, at their own charge or no charge, with the information providing company to become, in advance, members who are to be supplied with medical information from the company; respective identification codes ID of the members; and respective pass codes of the members. The memory device 28 additionally includes a medical-information DB (data base) 32 that stores respective sets of medical information including respective sets of physical information sent from the members, and respective medical evaluations or diagnoses obtained based on those sets of physical information. The memory device 28 further includes a virtual-doctor DB (data base) or a health secretary DB 34 that stores a diagnosing program for use in automatically making those medical advices, evaluations or diagnoses based on the sets of physical information or respective changes (or trends) of the sets of physical information.

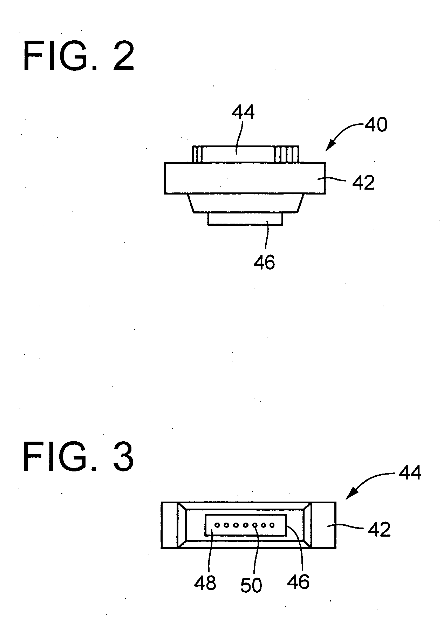

Each of the portable telephones 10 includes well-known display 36 and keys 38, and a sensor 40 functioning as a physical-information sensor (i.e., a physical-information obtaining device) is inserted in a connector, not shown,, provided in a bottom portion of the telephone 10, so that the sensor 40 is directly connected to the telephone 40. As shown in detail in Figs. 2 and 3, the sensor 40 includes a main body 42, a connector 44 that projects from the main body 42 toward the telephone 10, and a sensing portion 46 that projects from the main body 42 in a direction opposite to the telephone 10. A free end surface of the sensing portion 46 provides a press surface 48 in which a plurality of pressure sensing elements 50 arranged, e.g., in an array are embedded. In addition to, or in place of, the array of pressure sensing elements 50, the press surface 48 may support a light emitting element and a light receiving element that cooperate with each other to measure a blood oxygen saturation, SaO₂, a photoelectric pulse wave, and a pulse rate; a temperature measuring element that measures a temperature, TB, of a living person; or one of a plurality of electrodes that cooperate with each other to detect an electrocardiographic waveform, ECG.

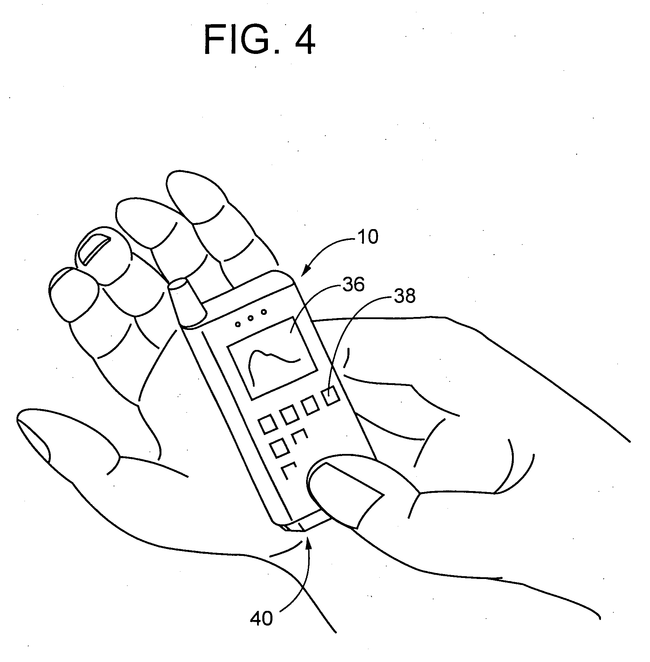

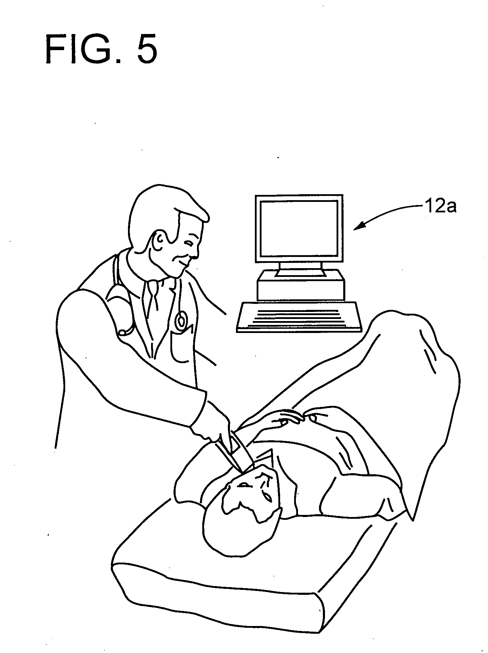

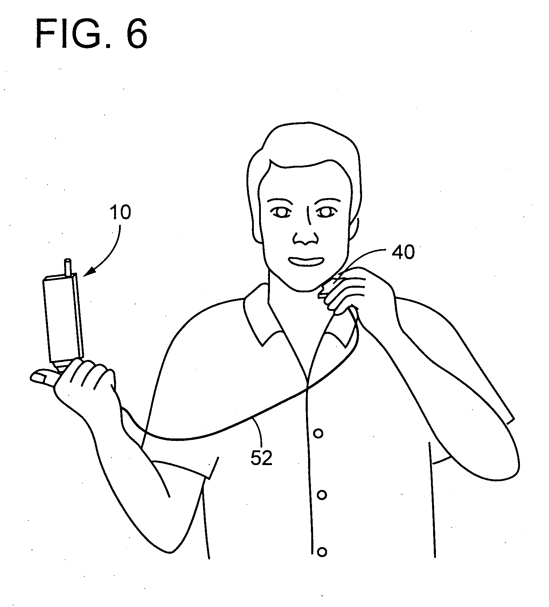

Fig. 4 shows a state in which the sensor 40 connected to each portable telephone 10 is pressed against a radial artery of a wrist of a living person so as to detect a pressure waveform from the radial artery, and the display 36 of the telephone 10 displays the thus detected pressure waveform. Fig. 5 shows a state in which a medical worker (e.g., a doctor or a specialist) presses a sensor 40 connected to a portable telephone 10, against a carotid artery of a patient so as to detect a carotid pressure waveform, and a display 36 of the telephone 10 or a display of, e.g., the medical worker's terminal device 12a displays the thus detected pressure waveform. The sensor 40 may be connected indirectly, e.g., via an electric line 52 to a connector, not shown, provided in the bottom portion of each portable telephone 10. Fig. 6 shows a state in which a portable telephone 10 is held by one of two hands of a living person and a sensor 40 that is connected via an electric line 52 to the telephone 10 and is held by the other hand is pressed against a carotid artery of the person so as to detect a carotid pressure waveform, and a display 36 of the telephone 10 displays the thus detected pressure waveform.

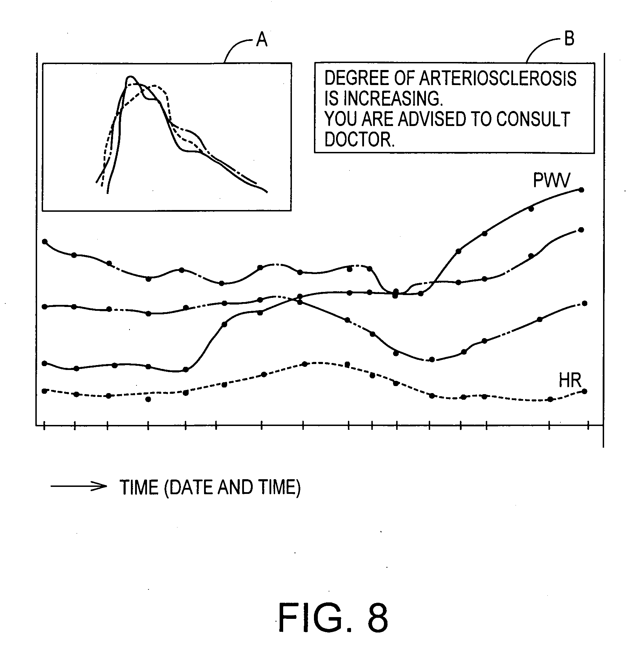

The portable telephones 10n are operated by the respective members who are registered in the server 14, so that each one of the telephones 10n periodically sends a set of physical information of a corresponding one of the members to the server 14. At that time, the server 14 stores the current set of physical information, and automatically makes, according to the diagnosing program stored in the virtual-doctor DB 34, a medical evaluation or diagnosis based on the current set of physical information or the change or trend of the current set of physical information from the past sets of physical information stored in the medical-information DB 32. In addition, the server 14 sends the thus made medical evaluation or diagnosis, together with the current and past sets of physical information of the member, to the member's telephone 10n, so that the medical evaluation or diagnosis and the sets of physical information are displayed on the display 36 of the telephone 10n. Each of the medical worker's terminal devices 12n may be operated by a corresponding one of the respective medical workers so as to request the server 14, using a pass code given to a member, to send the set of medical information related to the member and stored in the medical-information DB 32. The server 14 retrieves, from the medical-information DB 32, the set of medical information corresponding to the pass code, and sends the thus obtained set of medical information to the terminal device 12n of the medical worker who has requested, so that the set of medical information is displayed on the display of the terminal device 12n. Fig. 8 shows an example of the set of medical information displayed by the terminal device 12n.

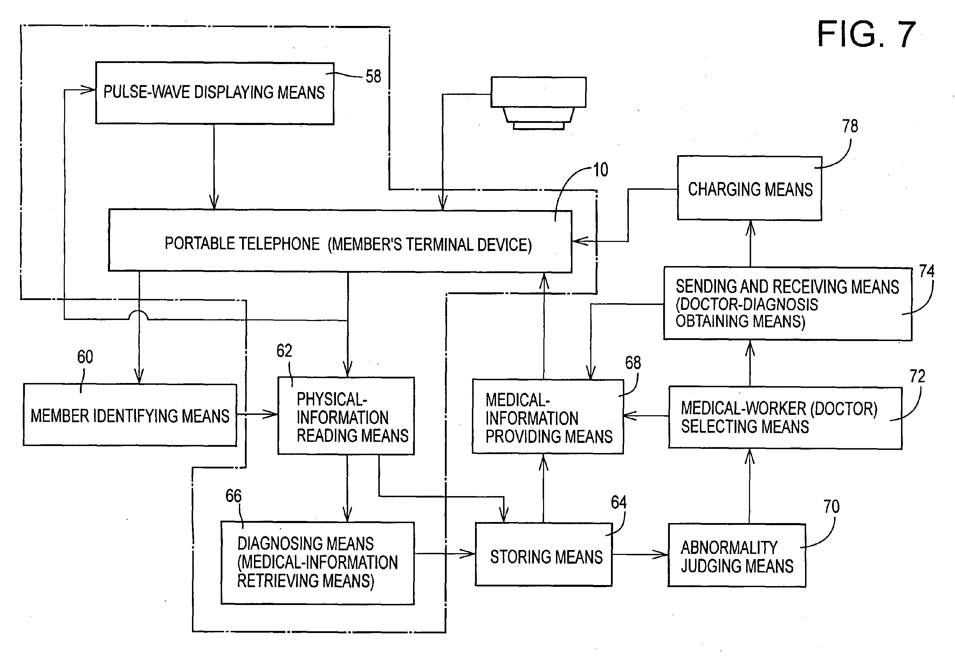

Fig. 7 is a block diagram for explaining essential control functions of the above-described server 14. A pulse-wave displaying device or means 58 operates, when receiving, from the sensor 40 connected to the portable telephone 10 either directly or indirectly via the electric line 52, a pressure-pulse-wave signal representing a pressure pulse wave produced from an artery present under a skin of a living person, the display 36 of the telephone 10 to display the pressure pulse wave. A pulse wave shown in a pulse-wave displaying area, A, at left above in Fig. 8 is identical with a pulse wave displayed by the display 36 of the telephone 10. The pulse-wave displaying area A displays, in addition to the pulse wave currently detected and normalized, one or more pulse waves that was or were detected and normalized at one or more prior times, and a reference pulse wave (i.e., a standard pulse wave) that is determined in advance. A member identifying device or means 60 judges whether an identification code ID inputted and sent by an arbitrary one of the portable telephones 10 is identical with one of the identification codes ID pre-stored in the member DB 30, and thereby judges whether a living person who has made access to the server 14 is one of the members registered in the server 14. A physical-information reading device or means 62 reads, when the member identifying means 60 has identified the person as one of the registered members, the set of physical information sent by the portable telephone 10, and a storing device or means 64 stores, in the medical-information DB 32, the thus read set of physical information in such a manner that the set of physical information read is associated with the identification code ID of the member identified. A diagnosing device or means, i.e., a medical- information retrieving device or means 66 automatically makes a diagnosis based on the set of physical information read by the reading means 62, according to the diagnosing program pre-stored in the virtual-doctor DB 34. For example, the diagnosing means 66 judges whether the current set of physical information read by the reading means 62, or a change (i.e., a trend) of the sets of physical information accumulatively stored by the storing means 64 falls in a predetermined reference range, and thereby selects an appropriate one of a plurality of medical evaluations or diagnoses pre-stored in the virtual-doctor DB 34. The storing means 64 stores, in the medical-information DB 32, the thus made medical evaluation or diagnosis such that the medical evaluation or diagnosis is associated with the identification code ID or the set of physical information. A medical-information providing device or means 68 sends, to the member's portable telephone 10 that has sent the current set of physical information, the current and past sets of physical information, and the medical evaluation or diagnosis made based on those sets of physical information, all of which have been stored by the storing means 64 in the medical-information DB 32, so that the sets of physical information and the medical evaluation or diagnosis are displayed on the member's telephone 10.

An abnormality judging device or means 70 judges whether each set of physical information read by the reading means 62, or an amount of change of the set of physical information, falls outside a predetermined normal range and thereby judges whether each member is abnormal. The normal range may be identical with the above-described reference range. A doctor selecting device or means, i.e., a medical-worker selecting device or means 72 selects, when the abnormality judging means 70 judges based on the set of physical information that the member is abnormal, the most appropriate one of doctors who have been listed and stored in the memory device 28, based on the sort of the physical information judged as abnormal, and an address of the abnormal member, such that the selected doctor is specialized in the sort of the abnormality and is near to the address of the abnormal member. A doctor-diagnosis obtaining device or means, i.e., a sending and receiving device or means 74 sends the current set of physical information judged as abnormal, and the past sets of physical information, and the automatic evaluation or automatic diagnosis stored in the medical-information DB 32, to the doctor selected by the doctor selecting means 72, requests the doctor to make a diagnosis on the current set of physical information, and receives the diagnosis made by the doctor. The storing means 64 stores, in the medical-information DB 32, the diagnosis made by the doctor, such that the diagnosis is associated with the identification code ID or the physical information, and the medical-information providing means 68 sends the diagnosis to the member. Fig. 8 shows an example of the diagnosis that is displayed on the display 36 of the member's portable telephone 10 that has sent the set of physical information to the server 14. In this way, each member can send his or her own physical information via the member's portable telephone 10, so as to obtain the automatically made diagnosis and additionally obtain, when the physical information is abnormal, the diagnosis made by the actual doctor based on the physical information. A charging device or means 78 charges the member for obtaining the diagnosis made by the actual doctor.

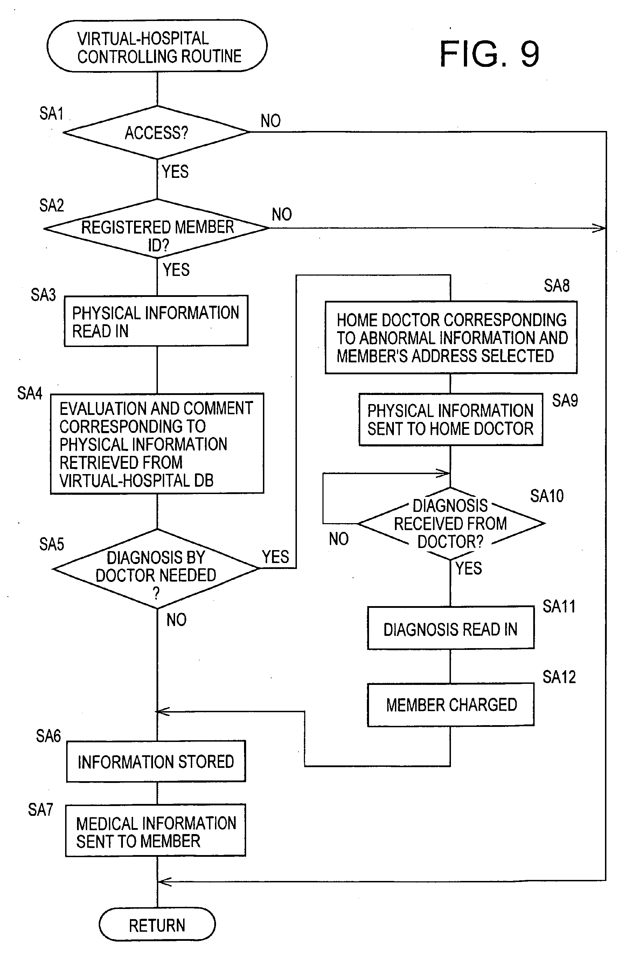

Fig. 9 shows a flow chart representing the essential control functions of the above-described server 14, i.e., a virtual-hospital controlling routine. This routine is iteratively executed at a short period, e.g., several milliseconds.

At Step SA1 of Fig. 9 (hereinafter, "Step" is omitted), the server judges whether any one of the portable telephones 10 has made access to the server. If a negative judgment is made at SA1, this routine is quitted. On the other hand, if a positive judgment is made at SA1, the control goes to SA2 corresponding to a member identifying step or the member identifying means 60. At SA2, the server judges whether the member's identification code ID inputted by the member's telephone is identical with one of the member's identification codes ID registered in the server. If a negative judgment is made at SA2, this routine is quitted. On the other hand, if a positive judgment is made at SA 2, the control goes to SA3 corresponding to a physical-information reading step or the physical-information reading means 62. At SA3, the server reads in a set of physical information that has been obtained by the sensor 40 connected to the telephone 10 and has been sent thereto from the telephone, i.e., a pressure pulse wave and, optionally, blood pressure BP, weight W, heart rate HR, electrocardiographic waveform ECG, temperature TB, autonomic-nerve activity, pulse-wave propagation velocity PWV, augmentation index AI, eye sight, blood sugar, presence or absence of allergy, and genes. One or more sorts of physical information that is or are not obtained by the sensor 40 is or are inputted through the keys 38 of the telephone 10.

Then, the control goes to SA4 corresponding to a diagnosing step or the diagnosing means, i.e., a medical-information retrieving step or the medical-information retrieving means 66. At SA4, the server automatically makes, according to the automatically evaluating and diagnosing program pre-stored in the virtual-doctor DB 34, a diagnosis on the new set of physical information, the past set or sets of physical information, and the change of the new and past sets of physical information. For example, the server selects and retrieves one of a plurality of evaluations and/or comments that corresponds to the new set of physical information. Fig. 8 shows the thus made evaluation (or comment) and the thus made virtual doctor's diagnosis (or comment) that are displayed in an evaluation displaying area, B. Then, the control goes to SA5 corresponding to an abnormality judging step or the abnormality judging means 70. At SA5, the server judges whether it is needed to consult an actual doctor or a specialist. More specifically described, the server judges whether the new set of physical information falls outside a predetermined normal range. If a negative judgment is made at SA5, the control goes to SA6 corresponding to a storing step or the storing means 64. At SA6, the server stores, in the medical-information DB 32, the new set of physical information and the automatically made evaluation and/or diagnosis, such that the set of medical information is associated with the identification code ID of the member. Then, the control goes to SA7 corresponding to a medical-information supplying step or the medical-information supplying means 68. At SA7, the server sends, to the member's portable telephone 10n that has sent the new set of physical information, the set of medical information that has been stored, for the member, in the medical-information DB 32, that is, the new and past sets of physical information and the automatically made evaluation and/or diagnosis, so that the set of medical information is displayed on the display of the member's telephone 10, as shown in Fig. 8. Thus, each member can easily and economically request the virtual hospital or doctor to check his or her physical condition or make a diagnosis on it.

On the other hand, if a positive judgment is made at SA5, the control goes to SA8 corresponding to a doctor selecting step or the doctor selecting means, i.e., a medical worker selecting step or the medical worker selecting means 72. At SA8, the server selects, from the pre-stored list of doctors, a doctor or a specialist who is appropriate for the sort of the abnormal physical information, the degree of abnormality of the physical information, and the address of the member. For example, a specialist who is specialized in the sort of the abnormal physical information, or a doctor belonging to a medical treatment facility located in an area where the member can go. Next, the control goes to SA 9 to SA11 corresponding to a doctor-diagnosis obtaining step or the doctor-diagnosis obtaining means, i.e., a sending and receiving step or the sending and receiving means 74. First, at SA9, the server sends, to the selected doctor, the abnormal, new set of physical information of the member and the past sets of physical information of the same, and requests the doctor to make a diagnosis on the sets of physical information. Then, at SA10, the server judges whether the server has received a diagnosis sent from the actual doctor. SA10 is repeated until a positive judgment is made. When a positive judgment is made at SA10, the control goes to SA11 to read in the diagnosis sent from the doctor. Then, at SA12, the server charges the member for obtaining the diagnosis made by the actual doctor. Subsequently, the control goes to SA6 and SA7. At SA6, the server stores, in the medical-information DB 32, not only the new set of physical information and the automatic evaluation or diagnosis, but also the diagnosis made by the actual doctor, such that the set of medical information is associated with the identification code ID of the member. Then, the control goes to SA7 corresponding to the medical-information providing step or the medical-information providing means 68. At SA7, the server sends, to the member's portable telephone 10 that has sent the new set of physical information, the set of medical information that has been stored, for the member, in the medical-information DB 32, that is, the new and past sets of physical information, the automatically made evaluation or diagnosis, and the diagnosis made by the actual doctor, so that the set of medical information is displayed on the member's telephone, as shown in Fig. 8, and additionally the diagnosis made by the actual doctor, and the name and address of the hospital to which the doctor belongs or the address where the doctor works are indicated in an area prepared therefor, not shown. Thus, each member can easily and economically request the virtual hospital or doctor to check his or her physical condition or make a diagnosis on it, and additionally can obtain a diagnosis made by the actual doctor when the new set of physical information is judged as abnormal. If necessary, the member can request the doctor to re-examine his or her physical condition.

As is apparent from the foregoing description of the illustrated embodiment, when the physical information obtained by the physical-information sensor 40 connected to the portable telephone 10 of a living person registered as a member is sent via the telephone 10 to the server 14, the memory device (i.e., the memory means) 28 of the server 14 stores the physical information for that member, and sends, to the member, the medical information corresponding to the thus stored physical information. Thus, even if the member may reside at a remote place, the member can automatically obtain the evaluation or diagnosis made on the physical information. That is, the member can quickly, economically and easily undergo a medical check-up via the communication line 16. In addition, since the medical information provided by the server 14 can indicate a sign of a serious disease, preventive medicine can be promoted. Since each member can quickly and easily obtain medical information, as needed, preventive medicine or early treatment can be improved, and a healthy family or home can be easily maintained.

Also, in the illustrated embodiment, the sensor (i.e., physical-information sensor) 40 is the pulse-wave sensor that is connected directly or indirectly to the portable telephone 10 and is pressed on the skin of the living person so as to detect the pulse wave produced by the artery present under the skin, and the telephone 10 incorporates the pulse-wave displaying means 58 that operates the display 36 to display the pulse wave detected by the sensor 40. Thus, the member can easily adjust, while viewing the pulse wave displayed on the display 36, the position at which the sensor 40 detects the pulse wave, or the pressure with which the sensor 40 is pressed.

Also, in the illustrated embodiment, the server 14 includes the medical-information data base 32 that stores the plurality of sets of medical information each of which includes the plurality of sorts of physical information obtained from a corresponding one of the members; and the diagnosing means (the medical-information retrieving means) 66 that retrieves, from the data base 32, the set of medical information to be provided to the one member, based on the set of physical information obtained by the sensor directly or indirectly connected to the portable telephone 10 of the one member and sent from the telephone. The medical-information providing means 68 provides the set of medical information retrieved by the medical-information retrieving means 66, to the portable telephone 10 of the one member. Thus, the medical information needed by each member can be automatically and economically provided to the each member.

Also, in the illustrated embodiment, the physical-information-abnormality judging means 70 (SA5) judges whether the set of physical information sent from each member falls outside the predetermined normal range and thereby judges whether the physical information is abnormal; the medical-worker selecting means 72 (SA8) selects, when the abnormality judging means 70 judges that the physical information sent from the each member is abnormal, one of the respective terminal devices 12n of the registered medical workers; and the sending and receiving means 74 (SA9 through SA11) sends the physical information judged as abnormal by the abnormality judging means 70, to the terminal device 12n of the medical worker selected by the medical-worker selecting means 72, requests the selected medical worker to make a diagnosis on the physical information, and receives the diagnosis made by the medical worker. The medical-information providing means 68 (SA7) sends the diagnosis sent from the terminal device of the medical worker and received by the sending and receiving means 74, to the terminal device of the member. Thus, when the physical information is abnormal, the member can automatically obtain the diagnosis made on the abnormal physical information by the actual doctor, i.e., highly reliable medical information.

Also, in the illustrated embodiment, the medical worker selecting means 72 (SA8) selects one of the medical workers based on the sort of physical information judged as being abnormal by the abnormality judging means 70 (SA5). The selecting means 72 selects, e.g., a specialist with respect to the field related to the abnormal physical information. Thus, when the physical information is abnormal, the member can obtain highly reliable medical information.

Also, in the illustrated embodiment, the medical worker selecting means 72 (SA8) selects one of the medical workers based on the address of the member who has sent the physical information judged as being abnormal by the abnormality judging means 70 (SA5). Thus, the selecting means 72 selects the medical worker appropriate for the address of the member, for example, a doctor who belongs to a medical treatment facility located in an area where the member can go from his or her address. Therefore, when the physical information is abnormal, the member can go to see the doctor who has made a diagnosis on the abnormal physical information.

Also, in the illustrated embodiment, when the medical-information providing means 68 (SA7) sends the diagnosis sent from the terminal device 12n of the medical worker, to the terminal device 10n of each member, the charging means 78 charges the each member for using the terminal device 10n. More specifically described, only the member who receives the diagnosis made on the abnormal physical information by the medical worker, i.e., only the beneficiary pays the cost needed for obtaining the additional diagnosis, and accordingly the subscription fee that each member must pay for routinely obtaining the evaluations or diagnoses automatically made on the respective sets of physical information by the medical-information data base, i.e., the so-called virtual health secretary or virtual hospital, that is, for usually using the e-health-care network, can be reduced. Thus, usually, each member can economically obtain a diagnosis made on physical information.

While the present invention has been described in its embodiment by reference to the drawings, it is to be understood that the present invention can otherwise be embodied.

For example, in the illustrated embodiment, the server 14 is described such that the server 14 includes the pulse-wave displaying means 58, the physical-information reading means 62, and the diagnosing means 66. However, those means 58, 62, 66 may be provided by the computer employed by each portable telephone 10. In the latter case, the telephone 10 includes the means 58, 62, 66 enclosed by a one-dot chain line in Fig. 7, and sends not only the physical information obtained by the sensor 40 but also a diagnosis made by the diagnosing means 66, to the server 14.

In addition, in the illustrated embodiment, the server 14 is described such that the server 14 is provided by a single computer. However, the server may be provided by a plurality of computers, and those computers may be provided at respective positions remote from each other.

Also, in the illustrated embodiment, the single sensor 40 directly or indirectly connected to each of the portable telephones 10 as the member's terminal devices has the functions of obtaining the blood pressure BP, the weight W, the heart rate HR, the electrocardiographic waveform ECG, the temperature TB, the autonomic-nerve activity, the pulse-wave propagation velocity PWV, the augmentation index AI, etc. However, the single sensor may be replaced with a plurality of exclusive sensors that detect the above-indicated sorts of physical information, respectively, and are independent of each other. One or more sorts of physical information that are not directly detected by the exclusive sensors may be manually inputted through each portable telephone 10 after having been measured using scales, a sphygmomanometer, a heart-rate meter, etc.

In the flow chart shown in Fig. 9, the order of the steps may be changed, as needed. In addition, SA8 to SA12 of Fig. 9 may be omitted.

While the present invention has been described in detail in its embodiments by reference to the drawings, it is to be understood that the present invention is not limited to the details of the described embodiments but may be embodied with various changes or improvements that may occur to a person skilled in the art.

## Claims

1. A medical-information providing apparatus comprising:
a physical-information sensor (40) which is directly or indirectly connected to a portable telephone (10) that is operated by a registered member, and which obtains physical information from the registered member;
a memory device (28, 64) to which the portable telephone is connected via a communication line (16) and which stores, for the registered member, the physical information obtained by the physical-information sensor; and
a medical-information providing device (68) which provides, to the registered member, medical information corresponding to the physical information stored for the registered member by the memory device.

2. A medical-information providing apparatus according to claim 1, wherein the physical-information sensor (40) comprises a pulse-wave sensor (46) which is adapted to be pressed against a skin of the registered member so as to detect a pulse wave produced by an artery present under the skin, and wherein the apparatus further comprises a pulse-wave displaying means (58) for operating a display device (36) of the portable telephone (10) to display the pulse wave detected by the pulse-wave sensor.

3. A medical-information providing apparatus according to claim 1 or claim 2, further comprising:
a medical-information data base (32) which stores a plurality of sets of medical information corresponding to a plurality of sets of physical information obtained from a plurality of registered members, respectively; and
a medical-information retrieving means (66) for retrieving, from the sets of medical information stored by the medical-information data base, a set of medical information to be provided to the registered member, based on a set of physical information obtained from the registered member by the physical-information sensor,
wherein the medical-information providing device (68) provides, to the portable telephone (10) operated by the registered member, the set of medical information retrieved by the medical-information retrieving means.

4. A medical-information providing apparatus according to any of claims 1 through 3, further comprising:
a physical-information-abnormality judging means (70) for judging whether the physical information sent from the portable telephone (10) operated by the registered member falls outside a predetermined reference range, and thereby judging whether the physical information is abnormal;
a medical-worker selecting means (72) for selecting, when the physical-information-abnormality judging means judges that the physical information is abnormal, one of a plurality of terminal devices (12), and thereby selecting one of a plurality of registered medical workers who operate the plurality of terminal devices, respectively; and
a sending and receiving means (74) for sending the physical information judged as being abnormal by the physical-information-abnormality judging means, to said one terminal device operated by said one medical worker selected by the medical-worker selecting means, requesting said one medical worker to make a diagnosis on the physical information judged as being abnormal, and receiving the diagnosis made by said one medical worker and sent from said one terminal device,
wherein the medical-information providing device (68) provides, to the portable telephone operated by the registered member, the set of medical information comprising the diagnosis sent from said one terminal device operated by said one medical worker and received by the sending and receiving means.

5. A medical-information providing apparatus according to claim 4, wherein the medical-worker selecting means (72) selects said one medical worker based on a sort of the physical information sent from the registered member and judged as being abnormal by the physical-information-abnormality judging means (70).

6. A medical-information providing apparatus according to claim 4 or claim 5, wherein the medical-worker selecting means (72) selects said one medical worker based on an address of the registered member who has sent the physical information to be judged as being abnormal by the physical-information-abnormality judging means (70).

7. A medical-information providing apparatus according to any of claims 4 through 6, further comprising a charging means (78) for charging the registered member operating the portable telephone (10), for the diagnosis sent by said one terminal device (12) operated by said one medical worker and provided by the medical-information providing device (68) to the portable telephone operated by the registered member.

8. A portable telephone (10, 40), operable by a member registered by a medical-information providing system (14), the telephone comprising:
a physical-information sensor (40) which obtains physical information from the registered member;
a diagnosing device (66) which automatically makes a diagnosis on the registered member based on the physical information obtained by the physical-information sensor; and
a sending device (10) which sends the physical information obtained by the physical-information sensor and the diagnosis made by the diagnosing means, to the medical-information providing system (14), so that the medical-information providing system provides medical information to the portable telephone.

9. A portable telephone according to claim 8, wherein the physical-information sensor (40) is adapted to be directly or indirectly connected to a connection potion of the portable telephone (10) and comprises at least one of a pressure-pulse-wave sensor (46) which is adapted to be pressed against the registered member so as to detect a pressure pulse wave produced by an artery of the member; a photoelectric-pulse-wave sensor; and a blood-oxygen-saturation sensor.

10. A portable telephone according to claim 8 or claim 9, wherein the diagnosing means (66) diagnoses, based on the physical information obtained by the physical-information sensor (40), the registered member with at least one of arrhythmia, condition of peripheral blood circulation, and decreasing of blood oxygen saturation.

11. A portable telephone according to any of claims 8 through 10, wherein the physical-information sensor (40) is adapted to be fixed on a skin of the registered member so as to obtain the physical information from a portion below the skin, and sends a detection signal representing the obtained physical information, to the portable telephone (10), and wherein the diagnosing means (66) makes the diagnosis on the registered member based on the physical information sent by the physical-information sensor.
